# EUROPÄISCHE PATENTSCHRIFT

(11) **EP 1 292 560 B1**
(45) Veröffentlichungstag und Bekanntmachung des Hinweises auf die Patenterteilung: **15.03.2006**
(21) Anmeldenummer: 01937911.4
(22) Anmeldetag: 14.06.2001
(51) Int. Cl.: C07C 67/03, C07C 69/732

(54) **VERFAHREN ZUR HERSTELLUNG VON HYDROXYPHENYLCARBONSÄUREESTERN**
METHOD FOR PREPARING HYDROXYPHENYL CARBOXYLIC ACID ESTERS
PROCEDE DE PRODUCTION D'ESTERS D'ACIDE HYDROXYPHENYLCARBOXYLIQUE

(30) Priorität: 23.06.2000 CH 125100; 22.02.2001 CH 312012001
(43) Veröffentlichungstag der Anmeldung: 19.03.2003
(73) Patentinhaber: Ciba Specialty Chemicals Holding Inc., 4057 Basel (CH)
(72) Erfinder: KLEINER, Christoph, CH-5073 Gipf-Oberfrick (CH)
(86) Internationale Anmeldenummer: PCT/CH2001/000370
(87) Internationale Veröffentlichungsnummer: WO 2001/098249

(56) Entgegenhaltungen:
- US-A- 4 716 244
- US-A- 5 563 291

## Beschreibung

Die Erfindung betrifft ein Verfahren zur Herstellung von Hydroxyphenylcarbonsäureestern unter Verwendung ausgewählter Katalysatoren. Die Erfindung betrifft auch neue Hydroxyphenylcarbonsäureester, welche gemäss dem erfindungsgemässen Verfahren hergestellt werden können.

Hydroxyphenylcarbonsäureester, insbesondere diejenigen der allgemeinen Formel (I), werden vorzugsweise als Antioxidantien eingesetzt. Zahlreiche Verbindungen der Formel (I) sind bekannt. Sie können bespielsweise durch Umesterung mit geeigneten Katalysatoren hergestellt werden. Solche Umesterungsverfahren sind z.B. in US-A-4,716,244; US-A-5,481,023; US-A-5,563,291 beschrieben.

GB-A.1081789 betrifft ebenfalls die Herstellung von Hydroxyphenylcarbonsäureestern mit Hilfe eines Umesterungsverfahrens, in dem Alkalimetallalkoholate als Katalysatoren eingesetzt werden.

EP-A-608089 beschreibt Ester des vorstehend genannten Strukurtyps, die Polyethylenglykolgruppen enthalten. Ihre Herstellung kann ebenfalls durch Umesterung erfolgen, wobei die bekannten stark basischen Katalysatoren wie Alkalimetalle sowie Alkalimetallamide, -alkoholate, -hydroxide und -(bi)carbonate empfohlen werden. Alle diese Verfahren erfordern stark alkalische Bedingungen und nach der Umsetzung einen Neutralisationsschritt.

Verbindungen der Formel (I) sind zum Teil wichtige Handelsprodukte. Sie schützen beispielsweise organische Materialien wie Kunststoffe und Schmierstoffe vor thermischem, oxidativem und/oder actinischem Abbau. Es besteht weiterhin Bedarf an neuen solchen Verbindungen für die Anwendung als Antioxydantien und an verbesserten Verfahren zu deren Herstellung.

Es wurde nun gefunden, dass man im an sich bekannten Umesterungsverfahren überraschenderweise ausgewählte Carbonsäuresalze von Alkalimetallen, wie beispielsweise Lithiumacetat, Natriumacetat oder Kaliumacetat, als Katalysatoren in praktisch neutralem Medium und ohne Lösungsmittel verwenden kann, wobei Produkte in Form von farblosen Schmelzen erhalten werden, welche direkt und ohne weitere Reinigungsverfahren eingesetzt werden können. Auch entfällt überraschenderweise die Neutralisation des Katalysators und Rückeaterungsreaktionen finden nicht statt, selbst wenn die ausreagierte abgekühlte Reaktionsmasse mit Alkoholen in Kontakt kommt. Bemerkenswert ist die Tatsache, dass die genannten Katalysatoren unter den angegebenen Bedingungen, insbesondere pH-neutral und ohne Lösungsmitteleinsatz, Umesterungen katalysieren, bei welchen n = 1,2,3,4 oder höher sein kann.

Die vorliegende Erfindung ist in den Ansprüchen definiert. Insbesondere betrifft die vorliegende Erfindung ein Verfahren zur Herstellung von Verbindungen der Formel (I) 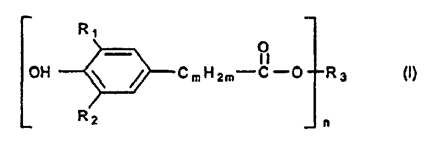
worin
- R₁ und R₂: unabhängig voneinander C₁-C₈-Alkyl, Cyclopentyl oder. Cyclohexyl,
- m: 1,2 oder 3, vorzugsweise 2,
- n: eine ganze Zahl von 1 bis 30, vorzugsweise eine ganze Zahl von 1 bis 10, insbesondere 1,2,3,4,5 oder 6, und vorzugsweise 1,2,3 oder 4,
- R₃: einen n-wertigen Rest von linearem oder verzweigtem C₄-C₃₀₋Alkyl, welches gegebenenfalls durch ein oder mehrere Sauerstoffatome unterbrochen ist, oder (für n = 1-12) einen n-wertigen Rest von C₅-C₁₂-Cycloalkyl, oder einen Rest R₄- [NR₅-CₘH₂ₘ-]ₚ,
- R₄: Wasserstoff, einen n-wertigen Rest von linearem oder verzweigtem C₄-C₃₀-Alkyl, welches gegebenenfalls durch eine oder mehrere Gruppen -NR₅- unterbrochen ist, oder(für n = 1-12) einen n-wertigen Rest von C₅-C₁₂-Cycloalkyl,
- R₅: unabhängig voneinander Wasserstoff oder Methyl oder -CₘH₂ₘ-, vorzugsweise Wasserstoff, und
- p: derjenigen Anzahl - [NR₅-CₘH₂ₘ-]-Gruppen entspricht, welche n Reste -CₘH₂ₘ- pro Molekül ergibt,
durch Umsetzung einer Verbindung der Formel (II) 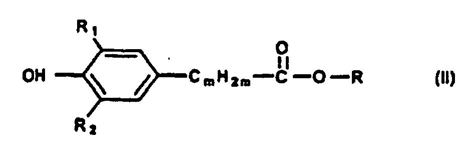
worin R C₁-C₃-Alkyl bedeutet,
mit einer Verbindung der Formel (III)

R₃ (OH)ₙ (III)

worin R₃, und n die oben angegebenen Bedeutungen haben,
dadurch gekennzeichnet, dass die Umsetzung bei praktisch neutralem pH-Wert und in Gegenwart von mindestens einem, im Reaktionsgemisch gelösten oder suspendierten, Alkalisalz einer organischen Carbonsäure, oder einem Gemisch solcher Alkalisalze, stattfindet, wobei (i) dieses Alkalisalz aus einem Alkalikation und einem Anion einer organischen Carbonsäure gebildet ist und (ii) die organische Carbonsäure bei den angewandten Reaktionsbedingungen zumindest teilweise flüchtig ist.

Dabei sind die bei der Umsetzung angewandten Reaktionsbedingungen vorzugsweise Temperaturen im Bereich von 50°C bis 250°C, vorzugsweise 80°C bis 220°C, vorzugsweise 140°C bis 220°C und Drucke im Bereich von 0.1 mbar bis 1 atm (Normaldruck), vorzugsweise 0.1 mbar bis 100 mbar, insbesondere 0.1 mbar bis 50 mbar und besonders 0.1 mbar bis 20 mbar. Vorzugsweise haben die Salz bildenden Carbonsäuren einen Siedepunkt, welcher innerhalb der genannten Temperatur- und Druckbereiche liegt.

Als Salz bildende Kationen von Alkalimetallen kommen insbesondere Lithium-, Natrium- oder Kaliumkationen in Frage. Bevorzugt sind Natrium-, Kalium- und/oder Lithiumkationen bzw. Natrium-, Kalium- und/oder Lithiumsalze von organischen Carbonsäuren.

Beispiele von Carbonsäuren, welche bei den angewandten Reaktionsbedingungen in ihrer Säureform zumindest teilweise flüchtig sind, sind aliphatische gesättigte oder ungesättigte Carbonsäuren mit vorzugsweise 2 bis 10 C-Atomen, vorzugsweise mit 2 bis 6 C-Atomen, wie z.B. Ameisensäure, Essigsäure, Propionsäure, n-Buttersäure, Isobuttersäure, n-Valeriansäure, Trimethylessigsäure, Capronsäure, n-Heptylsäure oder Pelargonsäure. Weitere Beispiele sind Malonsäure, Maleinsäure, Fumarsäure oder auch Malonsäuremonomethylester. Geeignet sind auch halogenierte Säuren, beispielsweise Fluoressigsäure, Chloressigsäure, Bromessigsäure, Difluoressigsäure, Dichloressigsäure, Trifluoressigsäure, Trichloressigsäure, alfa-Chlorpropionsäure oder beta-Chorpropionsäure. Bevorzugt sind Natriumacetat, Kaliumacetat, Lithiumacetat, Natriumformiat, Kaliumformiat oder Lithiumformiat oder ein Gemisch dieser Verbindungen.

Der Katalysator wird vorzugsweise in Mengen von 0.05 bis 5 Mol%, bezogen auf die umzusetzende Molmenge der Verbindung der Formel (I), zugegeben.

R₁ bedeutet vorzugsweise C₁-C₈-Alkyl, wobei dieser Alkylrest linear oder verzweigt sein kann. Vorzugsweise bedeutet R₁ einen Alkylrest mit 1-4 C-Atomen, vorzugsweise Methyl, Ethyl, Propyl oder Butyl. Vorzugsweise bedeutet R₁ einen verzweigten Rest, vorzugsweise Methyl oder tert.-Butyl.

R₂bedeutet vorzugsweise C₁-C₈-Alkyl, wobei dieser Alkylrest linear oder verzweigt sein kann. Vorzugsweise bedeutet R₂ einen Alkylrest mit 1-4 C-Atomen, vorzugsweise Methyl, Ethyl, Propyl oder Butyl. Vorzugsweise beutet R₂ einen verzweigten Rest, vorzugsweise Methyl oder tert.-Butyl, vorzugsweise tert.-Butyl.

Verbindungen der Formel (I) können in demselben Molekül mit R₁ und R₂ in unterschiedlicher Weise substituierte Phenylreste enthalten, und R₁ und R₂ an demselben Phenylrest können gleich oder verschieden sein. In diesem Sinne können z.B. R₁ und R₂ beide Methyl oder tert.-Butyl, oder R₁ Methyl und R₂ tert.-Butyl, bedeuten.

R₃ als n-wertigen Alkylrest mit 4-30 C-Atomen, welcher gegebenenfalls durch Sauerstoff unterbrochen ist, kann linear oder verzweigt sein oder als Gemisch vorliegen. Solche Gemische können aus Verbindungen bestehen, in welchen R₃ ein Gemisch von vorwiegend linearen oder verzweigten Alkylresten darstellt, beispielsweise mit 14, 16, 18 und 20 C-Atomen, wobei diese Alkylreste teilweise auch verzweigt sein können.

Bedeutet R₃ einen Rest R₄-[NR₅-CₘH₂ₘ-]ₚ, so bedeutet dieser Rest vorzugsweise R₄- [NH-CₘH₂ₘ-]ₚ oder R₄- [N(CₘH₂ₘ-)₂]ₚ und vorzugsweise R₄- [NH-CₘH₂ₘ-]ₚ. Darin ist R₄ vorzugsweise ein Alkylrest mit 4-30 C-Atomen, welcher gegebenenfalls durch -NR₅-, vorzugsweise -NHunterbrochen ist, und linear oder verzweigt sein kann, beispielsweise mit 14, 16, 18 und 20 C-Atomen. Ebenso kann R₄ abgeleitet sein (für n=2) von Alkylendiamin, wie Ethylendiamin oder Propylendiamin oder von einem Polyalkylendiamin, vorzugsweise einem Dialkylendiamin wie Diethylendiamin oder Dipropylendiamin..

Bedeutet n=1, so bedeutet R₃ vorzugsweise einen einwertigen Rest, vorzugsweise n-Butyl, Isobutyl, tert.-Butyl, Pentyl, Isopentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Eicosyl sowie der entsprechenden homologen Resten mit steigenden C-Atomen bis 30 C-Atomen oder ein Gemisch dieser C₄-C₃₀-Reste. Für n=1 bedeutet R₃ vorzugsweise auch einen einwertigen Rest wie Cycloalkyl mit 5-12 C-Atomen, vorzugsweise Cyclopentyl und Cyclohexyl, insbesondere Cyclohexyl. Bevorzugt bedeutet R₃ (für n=1) einen einwertigen C₈₋C₃₀-Alkylrest, und vorzugsweise i-Octyl, 2-Ethylhexyl, n-Hexadecyl und n-Octadecyl oder ein Gemisch dieser Alkylreste. Diese hier genannten Bedeutungen für R₃ sind auch bevorzugte Bedeutungen für R₄.

Bedeutet n=2, so bedeutet R₃ vorzugsweise C₂-C₈-Alkylen oder durch Sauerstoff unterbrochenes C₄-C₁₂-Alkylen. In diesem Sinne stellt R₃ (für n=2) eine, durch Weglassen der beiden OH-Gruppen von einem zweiwertigen Alkohol abgeleitete, Alkylengruppe dar, welche durch Sauerstoff unterbrochen sein kann. Beispiele für solche zweiwertigen Alkohole sind Alkylenglykole, wie 1,2-Ethandiol, 1,3-Propandiol, 1,4-Butandiol, 1,5-Pentandiol, 1,6-Hexandiol, 1,7-Heptandiol, 1,8-Octandiol, Polyalkylenglykole wie Diethylenglycol, Triethylenglycol und Tetraethylenglycol, Dipropylenglykol, Tripropylenglykol, oder (für n=1) Glycerin, Pentaerythit [C(CH₂OH)₄] oder analoge Polyole. Ebenso können die n-wertigen Reste R₃ welche durch Sauerstoff unterbrochen sind, abgeleitet sein von verätherten Polyhydroxyverbindungen, vorzugsweise von Polyglycerin oder von Polypentaerythit.

Analogerweise stellt R₄ (für n=2) vorzugsweise eine, durch Weglassen der beiden H₂N-Gruppen von einem Diamin abgeleitete, Alkylengruppe dar, welche durch -NR₅-, vorzugsweise -NH-, unterbrochen sein kann. Beispiele für solche Diamine sind Alkylendiamine, wie 1,2-Ethylendiamin oder 1,2-Propylendiamin oder Dialkylendiamine wie Di(ethylendiamin) oder Di(propylendiamin). Di(ethylendiamin) beispielsweise kann am mittleren Stickstoffatom gegebenenfalls eine [-NH-CₘH₂ₘ-]-Gruppe aufweisen.

Bedeutet n=3, so ist R₃ eine durch Weglassen der OH-Gruppen von einem 3-wertigen Alkohol abgeleitete verzweigte Alkyl-Alkylengruppe. In diesem Sinne bedeutet R₃ vorzugsweise Methan-C₁-C₆₋Alkyl-trimethylen. Beispiele für solche 3-wertige Alkohole sind 1,1,1-tris-Hydroxymethylethan (Trimethylolethan), 1,1,1-tris-Hydroxymethylpropan (Trimethylolpropan).

Bedeutet n=4, so ist R₃ eine durch Weglassen der OH-Gruppen von einem 4-wertigen Alkohol bzw. Polyol abgeleitete verzweigte Alkyl-Alkylengruppe. In diesem Sinne bedeutet R₃ vorzugsweise Methantetramethylen, beispielsweise Tetrakis-hydroxymethylmethan (Pentaerithryt).

Im Alkylenrest-(CₘH₂ₘ) - der Verbindung der Formel (I) resp. der Formel (II) bedeutet m vorzugsweise zwei, bzw. der Alkylenrest bedeutet -CH₂CH₂-.

Besonders bevorzugt werden Verbindungen der Formel (I) hergestellt, worin R₁ tert.-Butyl, und R₂ Methyl oder tert.-Butyl, und R₃ n-Octadecyl, ein Gemisch von höheren Alkylresten mit 8-30 C-Atomen (C₈-C₃₀-Aylreste) oder einen von 1,6-Hexandiol, Triethylenglycol, Pentaerithryt, 1,1,1-tris-Hyroxymethylethan, 1,1,1-tris-Hydroxymethylpropan, oder R₄ einen von 1,2-Ethylendiamin oder 1,2-Propylendiamin oder wie Di(ethylendiamin) oder Di(propylendiamin)abgeleiteten Rest darstellen.

Die Erfindung betrifft auch die Verwendung eines Alkalisalzes einer organischen Carbonsäure, oder eines Gemisches solcher Alkalisalze, wie dies vorgehend beschrieben ist, als Katalysator bei der Herstellung von Verbindungen der Formel (I), ausgehend von Verbindungen der Formel (II) und (III), wie dies vorgängig beschrieben ist, wobei die Verwendung von Natriumacetat, Kaliumacetat, Lithiumacetat, Natriumformiat, Kaliumformiat oder Lithiumformiat oder ein Gemisch dieser Verbindungen, bevorzugt ist.

Der Katalysator wird in Mengen von 0.05 bis 5 Mol%, bevorzugt 0.05 bis 3 Mol%, besonders bevorzugt 0.1 bis 1 Mol%, bezogen auf die umzusetzende Molmenge der Verbindung der Formel (I), zugegeben.

Bevorzugt wird erfindungsgemäss ohne Lösungsmittel gearbeitet. Es ist jedoch auch möglich die Umesterungsreaktion erfindungsgemäss in einem inerten organischen aliphatischen und/oder aromatischen Lösungsmittel oder einem Gemisch solcher Lösungsmittel durchzuführen.

Erfindungsgemäss werden die Verbindungen der Formeln (II) und (III) zusammen mit dem Katalysator unter inerten Bedingungen, vorzugsweise bei einer Temperatur von 90-120°C und unter Rühren, erhitzt, bis eine Schmelze vorliegt. Zur Verschiebung des chemischen Gleichgewichts in der Umesterungsreaktion wird anschliessend der Druck reduziert und die Temperatur erhöht, das heisst, die Reaktion wird vorzugsweise bei einem Druck im Bereich von 0.1-200 mbar und bei Temperaturen im Bereich von 140-220°C durchgeführt. Der Druck liegt bevorzugt bei 0.1-50 mbar, besonders bevorzugt bei 0.1-20 mbar. Die Reaktionstemperatur liegt bevorzugt bei 160-220°C, besonders bevorzugt bei 165-185°C.

Die Reaktionszeit ist abhängig von Druck und der Temperatur und liegt im allgemeinen zwischen 1 und 12 Stunden, vorzugsweise zwischen 1 bis 10 Stunden, insbesondere zwischen 2 bis 6 Stunden.

In der Regel setzt man einen kleinen Überschuss des Esters der Formel (II) bezogen auf die Hydroxyfunktionen des Alkohols der Formel (III) ein. Vorzugsweise liegt das Verhältnis der Verbindung der Formel (II) zu der Verbindung der Formel (III) [berechnet in Moläquivalenten] im Bereich von 0.8:1 bis 1.5:1, bevorzugt im Bereich von 1:1 bis 1.2:1, besonders bevorzugt zwischen 1.05:1 und 1.15:1.

Wird ein Überschuss der Verbindung der Formel (II) eingesetzt, so wird dieser am Ende der Reaktion vorzugsweise abdestilliert, wobei dieser Überschuss dann auch als Schlepper für allenfalls entstandene unerwünschte, die Farbe des Produktes (I) beeinträchtigende, Nebenkomponenten dient.

Das Produkt der Formel (I) kann direkt durch Abkühlen, mit oder ohne Animpfen, zur Kristallisation oder Erstarrung gebracht werden, worauf es ohne zusätzlichen Reinigungsschritt, wie beispielsweise einer Rekristallisation, direkt zur Handelsform weiterverarbeitet werden kann. Erfindungsgemäss kann die Schmelze natürlich auch in einem geeigneten Lösungsmittel aufgenommen, abgekühlt und, mit oder ohne Animpfen, kristallisiert werden. Als Lösungsmittel geeignet sind beispielsweise aliphatische Kohlenwasserstoffe, wie Heptan oder Cyclohexan oder Mischungen derselben; aromatische Kohlenwasserstoffe, wie Toluol oder/oder Xylol; Alkohole wie Methanol, Ethanol, Propanol und/oder Isopropanol sowie die entsprechenden Alkohol/Wasser-Gemische (50-100% Alkohol). Bevorzugt sind Methanol und/oder Isopropanol und deren Gemischen mit Wasser.

Der Restgehalt an Katalysator im Produkt, beispielsweise vor oder nach allfälliger Filtration der ausreagierten Reaktionsschmelze, stört normalerweise bei der Verwendung als Stabilisator nicht. Vorhandene Alkalisalze können jedoch mittels einfacher Filtration, beispielsweise in an sich bekannter Weise über eine 20µ-Filterplatte bei 90°C bis 130°C, entfernt werden. Nach der Filtration liegt der Gehalt an Katalysator in der Regel im ppm-Bereich.

Besondere Vorteile des erfindungsgemässen Verfahrens liegen darin, dass das Produkt analysenrein, ohne störende Verfärbungen, das heisst ohne farbige oder verfärbende Nebenkomponenten in der Reaktionsschmelze bzw. den Produkten erhalten wird, keine weiteren Reinigungsschritte nötig sind und das Reaktionsprodukt ohne Zugabe von Zusatzstoffen, mittels physikalischen Methoden, z.B. mittels Vermahlen oder Pelletieren, in eine verwendungsfähige Handelsform gebracht werden kann.

Die im erfindungsgemässen Verfahren eingesetzten Verbindungen der Formeln (II) und (III) sind zum grossteil an sich bekannt. Die Verbindungen der Formel (I), worin n=3 und R₃ einen dreiwertigen Rest, der von 1,1,1-tris-Hydroxymethylethan (Trimethylolethan) abgeleitet ist, bzw. worin R₃ 1,1,1-tris-Methylenethan bedeutet, sind zum Teil neu. Ebenso sind die Verbindungen der Formel (I), worin n=3 und R₃ einen dreiwertigen Rest, der von 1,1,1-tris-Hydroxymethylpropan (Trimethylolpropan) abgeleitet ist, bzw. worin R₃ 1,1,1-tris-Methylenpropan bedeutet, zum Teil neu.

Die Verbindungen der Formel (I), worin und R₃ einen Rest R₄-[NR₅₋CₘH₂ₘ-]ₚ, vorzugsweise R₄-[NH-CₘHₘ-]ₚ bedeutet, worin R₄ einen von 1,2-Ethylendiamin oder 1,2-Propylendiamin oder von Di(ethylendiamin) oder Di(propylendiamin)abgeleiteten Rest bedeutet und R₁, R₂, und m die angegebenen Bedeutungen haben, sind neu.

Diese letzteren Verbindungen sind Gegenstand der vorliegenden Erfindung. Sie können auch mit andern an sich bekannten Herstellungsmethoden hergestellt werden und sind nicht an das in der vorliegenden Erfindung dargelegte Herstellungsverfahren gebunden. Überraschenderweise zeigen die Verbindungen in einer basischen acetonischen Lösung nur eine sehr geringe Tendenz zur Gelbfärbung, was einen wichtigen Hinweis auf applikatorische Vorteile in Bezug auf den Yellowness Index darstellt.

Die folgenden Beispiele erläutern die Erfindung weiter, ohne sie jedoch zu beschränken:

### Beispiel 1: β-(3,5-Di-t-butyl-4-hydroxyohenyl)pronionsäureoctadecylester (Verbindung der Formel (I), worin R₁ und R₂=tert.-Butyl, n=1, m=2, R₃=n-C₁₈H₃₇)

109 g (0.374 mol) β-(3,5-Di-t-butyl-4-hydroxyphenyl)propionsäuremethylester, 92.5 g (0.34 mol) Stearylalkohol und 0.1 g Lithiumacetat als Dihydrat (0.001 mol) werden vorgelegt und bei 100°C unter Stickstoff geschmolzen und verrührt. Sobald eine Schmelze vorliegt, wird vorsichtig evakuiert und gleichzeitig auf eine Innentemperatur (IT) von 150-160°C erwärmt. Das entstehende Methanol wird abdestilliert und in einer Kühlfalle kondensiert.
Sobald ein Innendruck von <10 mbar erreicht ist, wird die Innentemperatur (IT) auf 170-180°C erhöht und weiter bis <1 mbar evakuiert. Sobald IT=180°C und der Druck <1 mbar erreicht sind, wird eine Stunde gehalten, anschliessend wird während 1-2 Stunden bei IT=200-210°C der Überschuss an β-(3,5-Di-t-butyl-4-hydroxyphenylpropionsäuremethylester ausdestilliert. Die Reaktionsschmelze enthält nun <0.2% der Edukte und hat einen Gehalt >98% an β-(3,5-Di-t-butyl-4-hydroxyphenyl)propionsäureoctadecylester. Die Reaktionsschmelze wird gekühlt und zur Kristallisation stehen gelassen. Ausbeute 96.5%; Smp. 51°C.

### Beispiel 2: β-(3,5-Di-t-butyl-4-hydroxyphenyl)propionsäureoctadecylester (Verbindung der Formel (I), worin R₁ und R₂=tert.-Butyl, n=1, m=2, R₃=n-C₁₈H₃₇)

109 g (0.374 mol) β-(3,5-Di-t-butyl-4-hydroxyphenyl)propionsäuremethylester, 92.5 g (0.34 mol) Stearylalkohol und 0.2 g Natriumformiat (0.003 mol) werden vorgelegt und bei 100°C unter Stickstoff geschmolzen und verrührt. Sobald eine Schmelze vorliegt, wird vorsichtig evakuiert und gleichzeitig auf eine Innentemperatur von 150-160°C aufgeheizt. Das entstehende Methanol wird abdestilliert und in einer Kühlfalle kondensiert. Sobald ein Innendruck von <10 mbar erreicht ist, wird die Innentemperatur auf 170-180°C erhöht und weiter bis <1 mbar evakuiert. Sobald IT=180° und Druck<1 mbar erreicht ist, wird eine Stunde gehalten, anschliessend wird während 1-2 Stunden bei IT=200-210°C der Überschuss an β-(3,5-Di-t-butyl-4-hydroxyphenyl)propionsäuremethylester ausdestilliert. Die Reaktionsschmelze enthält nun <0.2% der Edukte und hat einen Gehalt >98% an β-(3,5-Di-t-butyl-4-hydroxyphenyl)propionsäureoctadecylester. Die Reaktionsschmelze wird gekühlt und zur Kristallisation stehen gelassen. Ausbeute 96.5%; Smp. 51°C.

### Beispiel 3: Triethylenglycyl-bis-[β-(3,5-Di-t-butyl-4-hydroxyphenyl)propionsäure]ester (Verbindung der Formel (I), worin R₁ und R₂=t-butyl , n=2, m=2 , R₃= -(CH₂CH₂O)₂CH₂CH₂)-)

76 g (0.26 mol) β-(3,5-Di-t-butyl-4-hydroxyphenyl)propionsäure-methylester, 15 g (0.1 mol) Triethylenglykol und 0.17 g Lithiumacetat als Dihydrat (0.0015 mol) werden vorgelegt und bei 100°C unter Stickstoff geschmolzen und verrührt. Sobald eine Schmelze vorliegt, wird vorsichtig evakuiert und gleichzeitig auf eine Innentemperatur von 160-180°C erwärmt. Das entstehende Methanol wird abdestilliert und in einer Kühlfalle kondensiert. Sobald ein Innendruck von <10 mbar erreicht ist, wird die Innentemperatur auf 180-190°C erhöht und weiter bis <1 mbar evakuiert. Sobald IT=190° und der Druck <1 mbar erreicht sind, wird eine Stunde gehalten, anschliessend wird während 1-2 Stunden bei IT=200-220°C der Überschuss an β-(3, 5-Di-t-butyl-4-hydroxyphenyl)propionsäuremethylester ausdestilliert. Die Reaktionsschmelze enthält nun <0.2% der Edukte und hat einen Gehalt >97% an Triethylenglycyl-bis-[β-(3,5-Di-t-butyl-4-hydroxyphenyl)propionsäure]ester. Die Reaktionsschmelze wird gekühlt und zur Kristallisation stehen gelassen. Ausbeute 94%; Smp. 113-115°C.

### Beispiel 4: Triethylenglycyl-bis-[β-(3-t-butyl-5-methyl-4-hydroxyphenyl)propionsäure]ester (Verbindung der Formel (I), worin R₁=tert.-Butyl, R₂=Methyl, n=2, m=2, R₃= (CH₂CH₂O)₂CH₂CH₂)-)

65 g (0.26 mol) β-(3-t-butyl-5-methyl-4-hydroxyphenyl)propionsäuremethylester, 15 g (0.1 mol) Triethylenglykol und 0.17 g Lithiumacetat als Dihydrat (0.0015 mol) werden vorgelegt und bei 100°C unter Stickstoff geschmolzen und verrührt. Sobald eine Schmelze vorliegt, wird vorsichtig evakuiert und gleichzeitig auf eine Innentemperatur von 160-170°C erwärmt. Das entstehende Methanol wird abdestilliert und in einer Kühlfalle kondensiert. Sobald ein Innendruck von <10 mbar erreicht ist, wird die Innentemperatur auf 170-180°C erhöht und weiter bis <1 mbar evakuiert. Sobald IT=180° und Druck <1 mbar erreicht sind, wird eine Stunde gehalten, anschliessend wird während 1-2 Stunden bei IT=190-200°C der Überschuss an β-(3-t-butyl-5-methyl-4-hydroxyphenyl)propionsäuremethylester ausdestilliert. Die Reaktionsschmelze enthält nun <0.2% der Edukte und hat einen Gehalt >97% an Triethylenglycyl-bis-[β-(3-t-butyl-5-methyl-4-hydroxyphenyl)propionsäure]ester. Die Reaktionsschmelze wird gekühlt und zur Kristallisation stehen gelassen. Ausbeute 94.5%; Smp. 74-77°C.

### Beispiel 5: 1,1,1-Tris-[β-(3,5-Di-t-butyl-4-hydroxyphenyl)-propionyloxymethyl]propan (Verbindung der Formel (I), worin R₁ und R₂=tert.-Butyl, n=3, m=2, R₃= CH₃CH₂C(CH₂)₃)

85.5 g (0.293 mol) β-(3,5-Di-t-butyl-4-hydroxyphenyl)propionsäuremethylester, 10.1 g (0.075 mol) 1,1,1-Tris-(hydroxymethyl)-propan und 0.23 g Lithiumacetat als Dihydrat (0.002 mol) werden vorgelegt und bei 120°C unter Stickstoff geschmolzen und verrührt. Sobald eine Schmelze vorliegt, wird vorsichtig evakuiert und gleichzeitig auf eine Innentemperatur von 160°C aufgeheizt. Das entstehende Methanol wird abdestilliert und in einer Kühlfalle kondensiert. Sobald ein Innendruck von <10 mbar erreicht ist, wird die Innentemperatur auf 180°C erhöht und weiter bis <1 mbar evakuiert. Sobald IT=180° und Druck<1 mbar erreicht sind, wird eine Stunde gehalten, anschliessend wird während 1-2 Stunden bei IT=200-210°C der Überschuss an β-(3,5-Di-t-butyl-4-hydroxyphenyl)propionsäuremethylester ausdestilliert. Die Reaktionsschmelze enthält nun <0.2% an β-(3,5-Di-t-butyl-4-hydroxyphenyl) propionsäure-methylester und hat einen Gehalt >97% an 1,1,1-Tris-[β-(3,5-Di-t-butyl-4-hydroxyphenyl)-propionyloxymethyl]propan. Die farblose Reaktionsschmelze wird filtriert, gekühlt und zur Erstarrung/Kristallisation gebracht. Ausbeute 95%; Smp. 66-79°C (amorphe Form)

### Beispiel 6: 1,1,1-Tris-(β-(3,5-Di-t-butyl-4-hydroxyphenyl)-propionyloxymethyl]propan (Verbindung der Formel (I), worin R₁ und R₂=tert .-Butyl , n=3, m=2, R₃= CH₃CH₂C(CH₂)₃)

85.5 g (0.293 mol) β-(3,5-Di-t-butyl-4-hydroxyphenyl)propionsäuremethylester, 10.1 g (0.075 mol) 1,1,1-Tris-(hydroxymethyl)-propan und 0.19 g Natriumacetat (0.002 mol) werden vorgelegt und bei 120°C unter Stickstoff geschmolzen und verrührt. Sobald eine Schmelze vorliegt, wird vorsichtig evakuiert und gleichzeitig auf eine Innentemperatur von 160°C erwärmt. Das entstehende Methanol wird abdestilliert und in einer Kühlfalle kondensiert. Sobald ein Innendruck von <10 mbar erreicht ist, wird die Innentemperatur auf 180°C erhöht und weiter bis <1 mbar evakuiert. Sobald IT=180° und Druck<1 mbar erreicht ist, wird eine Stunde gehalten, anschliessend wird während 1-2 Stunden bei IT=200-210°C der Überschuss an β-(3,5-Di-t-butyl-4-hydroxyphenyl)propionsäuremethylester ausdestilliert. Die Reaktionsschmelze enthält nun <0.2% an β-(3,5-Di-t-butyl-4-hydroxyphenyl) propionsäure-methylester und hat einen Gehalt >97% an 1,1,1-Tris-[β-(3,5-Di-t-butyl-4-hydroxyphenyl)-propionyloxymethyl]propan. Die farblose Reaktionsschmelze wird filtriert, gekühlt und zur Erstarrung/Kristallisation gebracht. Ausbeute 95.5%; Smp. 66-79°C (amorphe Form)

### Beispiel 7: 1,1,1-Tris-[?-(3,5-Di-t-butyl-4-hydroxyphenyl)propionyloxymethyl]ethan(verbindung der Formel (I), worin R₁ und R₂=tert. -Butyl, n=3, m=2 , R₃=CH₃C(CH₂)₃)

85.5 g (0.293 mol) β-(3,5-Di-t-butyl-4-hydroxyphenyl)propionsäuremethylester, 9 g (0.075 mol) 1,1,1-Tris-(hydroxymethyl)ethan und 0.23 g Lithiumacetat als Dihydrat (0.002 mol) werden vorgelegt und bei 120°C unter Stickstoff geschmolzen und verrührt. Sobald eine Schmelze vorliegt, wird vorsichtig evakuiert und gleichzeitig auf eine Innentemperatur von 160°C erwärmt. Das entstehende Methanol wird abdestilliert und in einer Kühlfalle kondensiert. Sobald ein Innendruck von <10 mbar erreicht ist, wird die Innentemperatur auf 180°C erhöht und weiter bis <1 mbar evakuiert. Sobald IT=180° und Druck<1 mbar erreicht ist, wird eine Stunde gehalten, anschliessend wird während 1-2 Stunden bei IT=200-210°C der Überschuss an β-(3,5-Di-t-butyl-4-hydroxyphenyl)propionsäuremethylester ausdestilliert. Die Reaktionsschmelze enthält nun <0.2% an β-(3,5-Di-t-butyl-4-hydroxyphenyl) propionsäure -methylester und hat einen Gehalt >97% an 1,1,1-Tris-(β-(3,5-Di-t-butyl-4-hydroxyphenyl)-propionyloxymethyl]ethan. Die farblose Reaktionsschmelze wird filtriert, gekühlt und zur Erstarrung/Kristallisation gebracht. Ausbeute 95%; Smp. 55-78°C (amorphe Form)

### Beispiel 8: Tetrakis-[β-(3,5-Di-t-butyl-4-hydroxyphenyl)propionyloxymethyl]methan (Verbindung der Formel (I), worin R₁ und R₂=tert.-Butyl, n=4, m=2, R₃=C(CH₂)₄)

75.9 g (0.26 mol) β-(3,5-Di-t-butyl-4-hydroxyphenyl)propionsäuremethylester, 6.8 g (0.05 mol) Pentaerythrit und 0.16 g Natriumacetat (0.002 mol) werden vorgelegt und bei 120°C unter Stickstoff geschmolzen und verrührt. Sobald eine Schmelze vorliegt, wird vorsichtig evakuiert und gleichzeitig auf eine Innentemperatur von 160-170°C erwärmt. Das entstehende Methanol wird abdestilliert und in einer Kühlfalle kondensiert. Sobald ein Innendruck von <10 mbar erreicht ist, wird die Innentemperatur auf 180°C erhöht und weiter bis <1 mbar evakuiert. Sobald IT=180°C und der Druck <1 mbar erreicht sind, wird eine Stunde gehalten, anschliessend wird während 1-2 Stunden bei IT=200-210°C der Überschuss an β-(3,5-Di-t-butyl-4-hydroxyphenyl)propionsäuremethylester ausdestilliert. Die Reaktionsschmelze enthält nun <0.2% an β-(3,5-Di-t-butyl-4-hydroxyphenyl)propionsäure-methylester und hat einen Gehalt >96% an Tetrakis-[β-(3,5-Di-t-butyl-4-hydroxyphenyl)-propionyloxymethyl]methan. Die farblose Reaktionsschmelze wird filtriert, gekühlt und zur Erstarrung/Kristallisation gebracht. Ausbeute 96%; Smp: 55-85°C (amorphe Form).

## Patentansprüche

1. Verfahren zur Herstellung von Verbindungen der Formel (I) 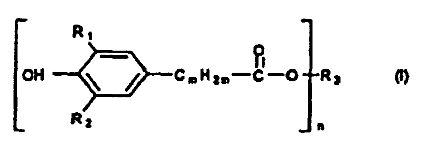
worin
R₁ und R₂ unabhängig voneinander C₁-C₈-Alkyl, Cyclopentyl oder Cyclohexyl,
m 1,2 oder 3,
n eine ganze Zahl von 1 bis 30, und
R₃ einen n-wertigen Rest von linearem oder verzweigtem C₄-C₃₀₋Alkyl, welches gegebenenfalls durch Sauerstoff unterbrochen ist, oder (für n = 1-12) einen n-wertigen Rest von C₅-C₁₂-Cycloalkyl, oder einen Rest R₄-[NR₅-CₘH₂ₘ-]ₚ,
R₄ Wasserstoff, einen n-wertigen Rest von linearem oder verzweigtem C₄-C₃₀-Alkyl, welches gegebenenfalls durch die Gruppe -NR₅- unterbrochen ist, oder(für n = 1-12) einen n-wertigen Rest von C₅-C₁₂-Cycloalkyl,
R₅ unabhängig voneinander Wasserstoff oder Methyl oder -CₘH₂ₘ-, und
p derjenigen Anzahl -[NR₅-CₘH₂ₘ-]-Gruppen entspricht, welche n Reste -CₘH₂ₘ- pro Molekül ergibt,
durch Umsetzung einer Verbindung der Formel (II) 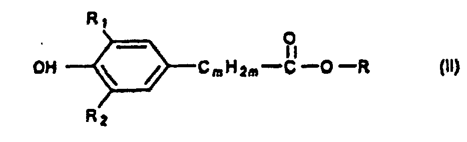
worin R C₁-C₃-Alkyl bedeutet,
mit einer Verbindung der Formel (III)
R₃ (OH)ₙ (III)
worin R₃ und n die angegebenen Bedeutungen haben, **dadurch gekennzeichnet, dass** die Umsetzung bei praktisch neutralem pH-Wert und in Gegenwart von mindestens einem, im Reaktionsgemisch gelösten oder suspendierten, Alkalisalz einer organischen Carbonsäure, oder einem Gemisch solcher Alkalisalze, stattfindet, wobei (i) dieses Alkalisalz aus einem Alkalikation und einem Anion einer organischen Carbonsäure gebildet ist und (ii) die organische Carbonsäure bei den angewandten Reaktionsbedingungen zumindest teilweise flüchtig ist.

2. Verfahren nach Anspruch 1, **dadurch gekennzeichnet, dass** das Alkalisalz der organischen Carbonsäure ein Natrium-, Kalium- und/oder Lithiumsalz darstellt.

3. Verfahren nach Anspruch 1 oder 2, **dadurch gekennzeichnet, dass** die Carbonsäure, aus welcher das Alkalisalz gebildet ist, eine aliphatische gesättigte oder ungesättigte Carbonsäuren mit vorzugsweise 2 bis 10 C-Atomen, vorzugsweise mit 2 bis 6 C-Atomen, darstellt.

4. Verfahren nach Anspruch 3, **dadurch gekennzeichnet, dass** die Carbonsäure aus der Gruppe Ameisensäure, Essigsäure, Propionsäure, n-Buttersäure, Isobuttersäure, n-Valeriansäure, Trimethylessigsäure, Capronsäure, n-Heptylsäure, Pelargonsäure, Malonsäure, Maleinsäure, Fumarsäure, Malonsäuremonomethylester, Fluoressigsäure, Chloressigsäure, Bromessigsäure, Difluoressigsäure, Dichloreasigsäure, Trifluoressigsäure, Trichloressigsäure, alfa-Chlorpropionsäure, und beta-Chorpropionsäure, ausgewählt ist.

5. Verfahren nach einem der Ansprüche 1-4, **dadurch gekennzeichnet, dass** als Katalysator Natriumacetat, Kaliumacetat, Lithiumacetat, Natriumformiat, Kaliumformiat oder Lithiumformiat oder ein Gemisch dieser Verbindungen verwendet werden.

6. Verfahren nach einem der Ansprüche 1-5, **dadurch gekennzeichnet, dass** R₁ lineares oder verzweigtes C₁-C₄-Alkyl, vorzugsweise Methyl, Ethyl, Propyl oder Butyl, vorzugsweise Methyl oder tert.-Butyl, bedeutet.

7. Verfahren nach einem der Ansprüche 1-6, **dadurch gekennzeichnet, dass** R₂ lineares oder verzweigtes C₁-C₄-Alkyl, vorzugsweise Methyl, Ethyl, Propyl oder Butyl, vorzugsweise tert.-Butyl, bedeutet.

8. Verfahren nach einem der Ansprüche 6 oder 7, **dadurch gekennzeichnet, dass** die Verbindung der Formel (I) in demselben Molekül mit R₁ und R₂ in unterschiedlicher Weise substituierte Phenylreste enthält.

9. Verfahren nach einem der Ansprüche 1-8, **dadurch gekennzeichnet, dass** R₄- [NR₅-CₘH₂ₘ-]ₚ einen Rest R₄-[NH-CₘH₂ₘ-]ₚ oder R₄₋[N(CₘH₂ₘ-)₂]ₚ und vorzugsweise R₄-[NH-CₘH₂ₘ-]ₚ bedeutet und R₄ von einem Alkylendiamin, vorzugsweise von Ethylendiamin oder Propylendiamin oder von einem Polyalkylendiamin, vorzugsweise einem Dialkylendiamin wie Diethylendiamin oder Dipropylendiamin, abgeleitet ist.

10. Verfahren nach einem der Ansprüche 1-8, **dadurch gekennzeichnet, dass** R₃ einen einwertigen Rest, vorzugsweise n-Butyl, Isobutyl, tert.-Butyl, Pentyl, Isopentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Eicosyl sowie der entsprechenden homologen Reste mit steigenden C-Atomen bis 30 C-Atomen oder ein Gemisch dieser C₄-C₃₀-Reste, oder einen einwertigen Rest, vorzugsweise Cyclopentyl und Cyclohexyl, bedeutet, vorzugsweise einwertiges i-Octyl, 2-Ethylhexyl, n-Hexadecyl und n-Octadecyl oder ein Gemisch dieser Alkylreste.

11. Verfahren nach einem der Ansprüche 1-8, **dadurch gekennzeichnet, dass** R₃ einen C₂-C₈-Alkylenrest oder durch Sauerstoff unterbrochenes C₄-C₁₂-Alkylen, darstellt, vorzugsweise einen, durch Weglassen der beiden OH-Gruppen von einem zweiwertigen Alkohol, abgeleiteten Rest bedeutet, vorzugsweise abgeleitet von Alkylenglykolen, vorzugsweise 1,2-Ethandiol, 1,3-Propandiol, 1,4-Butandiol, 1,5-Pentandiol, 1,6-Hexandiol, 1,7-Heptandiol, 1,8-Octandiol; von Polyalkylenglykolen vorzugsweise Diethylenglycol, Triethylenglycol und Tetraethylenglycol, Dipropylenglykol, Tripropylenglykol; von Glycerin, Pentaerytrit oder analogen Polyolen; von verätherten Polyhydroxyverbindungen, vorzugsweise von Polyglycerin oder Polypentaerythit.

12. Verfahren nach einem der Ansprüche 1-9, **dadurch gekennzeichnet, dass**, R₄ eine, durch Weglassen der beiden H₂N-Gruppen von einem Diamin, abgeleitete Alkylengruppe darstellt, welche gegebenenfalls durch -NR₅-, vorzugsweise durch -NH-, unterbrochen ist und vorzugsweise von einem Alkylendiamin, vorzugsweise 1,2-Ethylendiamin oder 1,2-Propylendiamin oder von einem Dialkylendiamin, vorzugsweise Di(ethylendiamin) oder Di(propylendiamin), abgeleitet ist.

13. Verfahren nach einem der Ansprüche 1-8, **dadurch gekennzeichnet, dass**, R₃ einen durch Weglassen der OH-Gruppen von einem 3-wertigen Alkohol (Polyol) abgeleitete verzweigte Alkyl-Alkylenrest bedeutet, vorzugsweise durch Weglassen der OH-Gruppen von 1,1,1-tris-Hydroxymethylethan (Trimethylolethan) und 1,1,1-tris-Hydroxymethylpropan (Trimethylolpropan).

14. Verfahren nach einem der Ansprüche 1-8, **dadurch gekennzeichnet, dass**, R₃ einen durch Weglassen der OH-Gruppen von einem 4-wertigen Alkohol abgeleiteten verzweigte Alkyl-Alkylenrest bedeutet, vorzugsweise Methantetramethylen.

15. Verfahren nach einem der Ansprüche 1-11, **dadurch gekennzeichnet, dass** in der Verbindung der Formel (I) R₁ tert.-Butyl, und R₂ Methyl oder tert.-Butyl, und R₃ n-Octadecyl, ein Gemisch von höheren Alkylresten mit 8-30 C-Atomen oder einen von 1,6-Hexandiol, Triethylenglycol, Pentaerithryt, 1,1,1-tris-Hyroxymethylethan oder 1,1,1-tris-Hydroxymethylpropan abgeleiteten Rest bedeuten.

16. Verfahren nach einem der Ansprüche 1-11, **dadurch gekennzeichnet, dass** in der Verbindung der Formel (I) R₁ tert.-Butyl, und R₂ Methyl oder tert.-Butyl, und R₄ einen von 1,2-Ethylendiamin oder 1,2-Propylendiamin oder von Di(ethylendiamin) oder Di(propylendiamin)abgeleiteten Rest darstellen.

17. Verfahren nach einem der Ansprüche 1-16, **dadurch gekennzeichnet, dass** die Verbindungen der Formeln (II) und (III) zusammen mit dem Katalysator unter inerten Bedingungen, bei einer Temperatur von 90-120°C erwärmt werden bis eine Schmelze vorliegt und die Umesterungsreaktion anschliessend bei reduziertem Druck im Bereich von 0.1-200 mbar und erhöhter Temperatur im Bereich von 140-220°C durchgeführt wird.

18. Verfahren nach einem der Ansprüche 1-17, **dadurch gekennzeichnet, dass** das Verhältnis der Verbindung der Formel (II) zu der Verbindung der Formel (III) [berechnet in Moläquivalenten] im Bereich von 0.8:1 bis 1.5:1, vorzugsweise im Bereich von 1:1 bis 1.2:1, und vorzugsweise zwischen 1.05:1 und 1.15:1, liegt.

19. Verfahren nach einem der Ansprüche 1-18, **dadurch gekennzeichnet, dass** das Produkt der Formel (I) durch Abkühlen, mit oder ohne Animpfen, zur Kristallisation oder Erstarrung gebracht und ohne zusätzlichen Reinigungsschritt direkt zur Handelsform weiter verarbeitet wird.

20. Verwendung von Alkalisalzen einer organischen Carbonsäure, oder von Gemischen solcher Alkalisalze, gemäss einem der Ansprüche 2-5, vorzugsweise von Natriumacetat, Kaliumacetat, Lithiumacetat, Natriumformiat, Kaliumformiat und Lithiumformiat oder einem Gemisch dieser Verbindungen, als Katalysator bei der Herstellung von Verbindungen der Formel (I) gemäss einem der Ansprüche 1-19.

21. Verwendung nach Anspruch 20, **dadurch gekennzeichnet, dass** der Katalysator in Mengen von 0.05 bis 5 Mol%, vorzugsweise 0.05 bis 3 Mol%, vorzugsweise 0.1 bis 1 Mol%, bezogen auf die umzusetzende Molmenge der Verbindung der Formel (I), anwesend ist.

22. Verbindungen der Formel (I), worin und R₃ einen Rest R₄₋[NR₅-CHₘH₂ₘ-]ₚ, vorzugsweise R₄-[NH-CₘB₂ₘ-]ₚ bedeutet, worin R₄ einen von 1,2-Ethylendiamin oder 1,2-Propylendiamin oder von Di(ethylendiamin) oder Di(propylendiamin)abgeleiteten Rest bedeutet und R₁, R₂, und m die Bedeutung gemäss Anspruch 1 haben.

## Claims

1. A process for the preparation of a compound of formula (I) 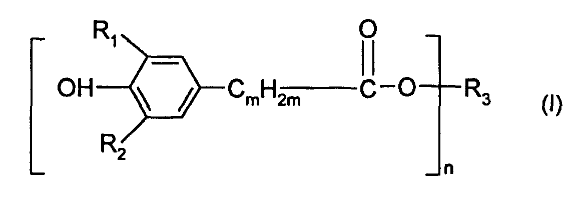
wherein
R₁ and R₂ are each independently of the other C₁-C₈alkyl, cyclopentyl or cyclohexyl,
m is 1, 2 or 3,
n is an integer from 1 to 30, and
R₃ is an n-valent radical from linear or branched C₄-C₃₀alkyl, which is uninterrupted or interrupted by oxygen, or (when n = 1-12) an n-valent radical from C₅-C₁₂cycloalkyl, or a radical R₄-[NR₅-CₘH₂ₘ-]ₚ,
R₄ is hydrogen, an n-valent radical from linear or branched C₄-C₃₀alkyl, which is uninterrupted or interrupted by the group -NR₅-, or (when n = 1-12) an n-valent radical from C₅-C₁₂cycloalkyl,
R₅ radicals, each independently of any other(s), are hydrogen or methyl or -CₘH₂ₘ-, and
p corresponds to the number of -[NR₅-CₘH₂ₘ-] groups that gives n -CₘH₂ₘ- radicals per molecule,
by reaction of a compound of formula (II) 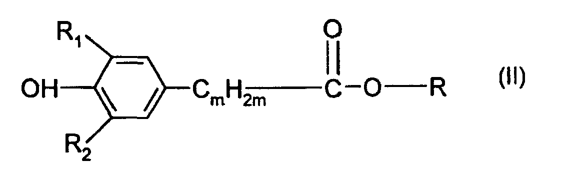 wherein R is C₁-C₃alkyl,
with a compound of formula (III)
R₃(OH)ₙ (III)
wherein R₃ and n are as defined above,
wherein the reaction takes place at practically neutral pH value and in the presence of at least one alkali metal salt of an organic carboxylic acid, which salt is dissolved or suspended in the reaction mixture, or in the presence of a mixture of such alkali metal salts, (i) that alkali metal salt being formed from an alkali metal cation and an anion of an organic carboxylic acid and (ii) the organic carboxylic acid being at least partially volatile under the reaction conditions applied.

2. A process according to claim 1, wherein the alkali metal salt of the organic carboxylic acid is a sodium, potassium and/or lithium salt.

3. A process according to claim 1 or claim 2, wherein the carboxylic acid from which the alkali metal salt is formed is an aliphatic, saturated or unsaturated carboxylic acid having preferably from 2 to 10 carbon atoms, especially from 2 to 6 carbon atoms.

4. A process according to claim 3, wherein the carboxylic acid is selected from the group formic acid, acetic acid, propionic acid , n-butyric acid, isobutyric acid, n-valeric acid, trimethylacetic acid, caproic acid, n-heptanoic acid, pelargonic acid, malonic acid, maleic acid, fumaric acid, malonic acid monomethyl ester, fluoroacetic acid, chloroacetic acid, bromoacetic acid, difluoroacetic acid, dichloroacetic acid, trifluoroacetic acid, trichloroacetic acid, α-chloropropionic acid and β-chloropropionic acid.

5. A process according to any one of claims 1 to 4, wherein sodium acetate, potassium acetate, lithium acetate, sodium formate, potassium formate or lithium formate or a mixture of such compounds is used as catalyst.

6. A process according to any one of claims 1 to 5, wherein R₁ is linear or branched C₁-C₄alkyl, preferably methyl, ethyl, propyl or butyl, especially methyl or tert-butyl.

7. A process according to any one of claims 1 to 6, wherein R₂ is linear or branched C₁-C₄alkyl, preferably methyl, ethyl, propyl or butyl, especially tert-butyl.

8. A process according to claim 6 or claim 7, wherein the compound of formula (I) contains phenyl radicals differently substituted by R₁ and R₂ in the same molecule.

9. A process according to any one of claims 1 to 8, wherein R₄-[NR₅-CₘH₂ₘ-]ₚ is a radical R₄-[NH-CₘH₂ₘ-]ₚ or R₄-[N(CₘH₂ₘ-)₂]ₚ, and especially R₄-[NH-CₘH₂ₘ-]ₚ and R₄ is derived from an alkylenediamine, preferably from ethylenediamine or propylenediamine, or from a polyalkylenediamine, preferably a dialkylenediamine, such as diethylenediamine or dipropylenediamine.

10. A process according to any one of claims 1 to 8, wherein R₃ is a monovalent radical, preferably n-butyl, isobutyl, tert-butyl, pentyl, isopentyl, hexyl, heptyl, octyl, 2-ethylhexyl, nonyl, decyl, undecyl, dodecyl, tridecyl, tetradecyl, pentadecyl, hexadecyl, heptadecyl, octadecyl, eicosyl or a corresponding homologous radical having carbon atoms increasing to 30 carbon atoms or a mixture of such C₄-C₃₀ radicals, or a monovalent radical, preferably cyclopentyl or cyclohexyl, preferably monovalent isooctyl, 2-ethylhexyl, n-hexadecyl or n-octadecyl or a mixture of such alkyl radicals.

11. A process according to any one of claims 1 to 8, wherein R₃ is a C₂-C₈alkylene radical, or C₄-C₁₂alkylene interrupted by oxygen, preferably a radical derived from a dihydric alcohol by omission of both OH groups, preferably derived from an alkylene glycol, preferably 1,2-ethanediol, 1,3-propanediol, 1,4-butanediol, 1,5-pentanediol, 1,6-hexanediol, 1,7-heptanediol or 1,8-octanediol; from a polyalkylene glycol, preferably diethylene glycol, triethylene glycol or tetraethylene glycol, dipropylene glycol or tripropylene glycol; from glycerol, pentaerythritol or an analogous polyol; or from an etherified polyhydroxy compound, preferably from polyglycerol or polypentaerythritol.

12. A process according to any one of claims 1 to 9, wherein R₄ is an alkylene group derived from a diamine by omission of both H₂N groups, which alkylene group is uninterrupted or interrupted by -NR₅-, preferably by -NH-, and preferably derived from an alkylenediamine, preferably 1,2-ethylenediamine or 1,2-propylenediamine, or from a dialkylenediamine, preferably di(ethylenediamine) or di(propylenediamine).

13. A process according to any one of claims 1 to 8, wherein R₃ is a branched alkylalkylene radical derived from a trihydric alcohol (polyol) by omission of the OH groups, preferably by omission of the OH groups from 1,1,1-tris-hydroxymethylethane (trimethylolethane) or from 1,1,1-tris-hydroxymethylpropane (trimethylolpropane).

14. A process according to any one of claims 1 to 8, wherein R₃ is a branched alkylalkylene radical derived from a tetrahydric alcohol by omission of the OH groups, preferably methanetetramethylene.

15. A process according to any one of claims 1 to 11, wherein, in the compound of formula (I), R₁ is tert-butyl and R₂ is methyl or tert-butyl, and R₃ is n-octadecyl, a mixture of higher alkyl radicals having from 8 to 30 carbon atoms or a radical derived from 1,6-hexanediol, triethylene glycol, pentaerythritol, 1,1,1-tris-hydroxymethylethane or 1,1,1-tris-hydroxymethylpropane.

16. A process according to any one of claims 1 to 11, wherein, in the compound of formula (I), R₁ is tert-butyl and R₂ is methyl or tert-butyl, and R₄ is a radical derived from 1,2-ethylenediamine or 1,2-propylenediamine or from di(ethylenediamine) or di(propylenediamine).

17. A process according to any one of claims 1 to 16, wherein the compounds of formulae (II) and (III) are heated together with the catalyst under inert conditions, at a temperature of from 90 to 120°C, until a melt is obtained and the transesterification reaction is then carried out at reduced pressure in the range from 0.1 to 200 mbar and at elevated temperature in the range from 140 to 220°C.

18. A process according to any one of claims 1 to 17, wherein the ratio of the compound of formula (II) to the compound of formula (III) [calculated in molar equivalents] is in the range from 0.8:1 to 1.5:1, preferably in the range from 1:1 to 1.2:1, and especially from 1.05:1 to 1.15:1.

19. A process according to any one of claims 1 to 18, wherein the product of formula (I) is caused to crystallise or solidify by cooling, with or without inoculation, and is further processed to its commercial form directly, without an additional purification step.

20. The use of an alkali metal salt of an organic carboxylic acid, or of a mixture of such alkali metal salts, according to any one of claims 2 to 5, preferably of sodium acetate, potassium acetate, lithium acetate, sodium formate, potassium formate or lithium formate or a mixture of such compounds, as a catalyst in the preparation of a compound of formula (I) according to any one of claims 1 to 19.

21. Use according to claim 20, wherein the catalyst is present in an amount of from 0.05 to 5 mol %, preferably from 0.05 to 3 mol %, especially from 0.1 to 1 mol %, based on the molar amount of the compound of formula (I) to be reacted.

22. A compound of formula (I) wherein and R₃ is a radical R₄-[NR₅-CₘH₂ₘ-]ₚ, preferably R₄-[NH-CₘH₂ₘ-]ₚ, wherein R₄ is a radical derived from 1,2-ethylenediamine or 1,2-propylenediamine or from di(ethylenediamine) or di(propylenediamine), and R₁, R₂ and m are as defined in claim 1.

## Revendications

1. Procédé de production de composés de formule (I) 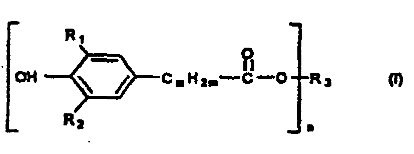
dans laquelle
R₁ et R₂ représentent, indépendamment l'un de l'autre, un groupe alkyle en C₁ à C₈, cyclopentyle ou cyclohexyle,
m vaut 1, 2 ou 3,
n représente un nombre entier allant de 1 à 30, et
R₃ représente un groupe n-valent d'un groupe alkyle en C₄ à C₃₀ linéaire ou ramifié, lequel est éventuellement interrompu par un atome d'oxygène, ou (si n = 1 à 12), un groupe n-valent d'un groupe cycloalkyle en C₅ à C₁₂, ou un groupe R₄-[NR₅-CₘH₂ₘ-]ₚ,
R₄ représente un atome d'hydrogène, un groupe n-valent d'un groupe alkyle en C₄ à C₃₀ linéaire ou ramifié, lequel est éventuellement interrompu par le groupe -NR₅-, ou (si n = 1 à 12) un groupe n-valent d'un groupe cycloalkyle en C₅ à C₁₂,
R₅ représente indépendamment un atome d'hydrogène ou un groupe méthyle ou -CₘH₂ₘ-, et
p correspond au nombre de groupes -[NR₅-CₘH₂ₘ-], lequel donne n groupes -CₘH₂ₘ- par molécule,
en faisant réagir un composé de formule (II) 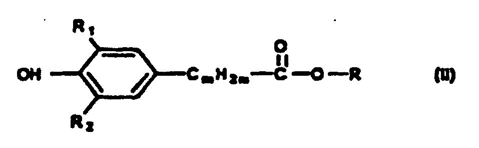
dans laquelle
R représente un groupe alkyle en C₁ à C₃,
avec un composé de formule générale (III)
R₃(OH)ₙ (III)
dans laquelle
R₃ et n ont les significations susmentionnées,
**caractérisé en ce que** la réaction a lieu à un pH pratiquement neutre et en présence d'au moins un sel alcalin d'un acide carboxylique, dissous ou mis en suspension dans le mélange réactionnel, ou dans un mélange de tels sels alcalins, dans lequel (i) ce sel alcalin est formé d'un cation alcalin et d'un anion d'un acide carboxylique organique et (ii) l'acide carboxylique organique est au moins partiellement volatil dans les conditions de la réaction mises en oeuvre.

2. Procédé selon la revendication 1, **caractérisé en ce que** le sel alcalin de l'acide carboxylique organique est un sel de sodium, de potassium et/ou de lithium.

3. Procédé selon la revendication 1 ou 2, **caractérisé en ce que** l'acide carboxylique à partir duquel le sel alcalin est formé est un acide carboxylique aliphatique saturé ou insaturé contenant de préférence 2 à 10 atomes de carbone, de préférence 2 à 6 atomes de carbone.

4. Procédé selon la revendication 3, **caractérisé en ce que** l'acide carboxylique est choisi dans le groupe formé par l'acide formique, l'acide acétique, l'acide propionique, l'acide n-butyrique, l'acide isobutyrique, l'acide n-valérique, l'acide triméthylacétique, l'acide caproïque, l'acide n-heptylique, l'acide pélargonique, l'acide malonique, l'acide maléique, l'acide fumarique, l'ester monométhylique de l'acide malonique, l'acide fluoroacétique, l'acide chloroacétique, l'acide bromoacétique, l'acide difluoroacétique, l'acide dichloroacétique, l'acide trifluoroacétique, l'acide trichloroacétique, l'acide α-chloropropionique et l'acide β-chloropropionique.

5. Procédé selon l'une quelconque des revendications 1 à 4, **caractérisé en ce que** l'on utilise, en tant que catalyseur, l'acétate de sodium, l'acétate de potassium, l'acétate de lithium, le formiate de sodium, le formiate de potassium ou le formiate de lithium ou un mélange des ces composés.

6. Procédé selon l'une quelconque des revendications 1 à 5, **caractérisé en ce que** R₁, représente un groupe alkyle en C₁ à C₄ linéaire ou ramifié, de préférence un groupe méthyle, éthyle, propyle ou butyle, de manière plus particulièrement préférée un groupe méthyle ou tert-butyle.

7. Procédé selon l'une quelconque des revendications 1 à 6, **caractérisé en ce que** R₂ représente un groupe alkyle en C₁ à C₄ linéaire ou ramifié, de préférence un groupe méthyle, éthyle, propyle ou butyle, de manière plus particulièrement préférée un groupe méthyle ou tert-butyle.

8. Procédé selon l'une quelconque des revendications 6 ou 7, **caractérisé en ce que** le composé de formule (I) contient, dans une même molécule, des groupes phényle substitués de manière différente par R₁ et R₂.

9. Procédé selon l'une quelconque des revendications 1 à 8, **caractérisé en ce que** R₄-[NR₅-CₘH₂ₘ-]ₚ est un groupe R₄-[NH-CₘH₂ₘ-]ₚ ou R₄-[N(CₘH₂ₘ-)₂]ₚ, et de préférence R₄-[NH-CₘH₂ₘ-]ₚ, et R₄ est dérivé d'une alkylènediamine, de préférence de l'éthylènediamine ou de la propylènediamine, ou d'une polyalkylènediamine, de préférence d'une dialkylènediamine telle que la diéthylènediamine ou la dipropylènediamine.

10. Procédé selon l'une quelconque des revendications 1 à 8, **caractérisé en ce que** R₃ représente un groupe monovalent, de préférence un groupe n-butyle, isobutyle, tert-butyle, pentyle, isopentyle, hexyle, heptyle, octyle, 2-éthylhexyle, nonyle, décyle, undécyle, dodécyle, tridécyle, tétradécyle, pentadécyle, hexadécyle, heptadécyle, octadécyle, eicosyle ainsi qu'un des groupes homologues correspondants contenant un nombre croissant d'atomes de carbone allant jusqu'à 30 atomes de carbone ou un mélange des ces groupes en C₄ à C₃₀, ou un groupe monovalent, de préférence un groupe cyclopentyle et cyclohexyle, de préférence un groupe i-octyle, 2-éthylhexyle, n-hexadécyle et n-octadécyle monovalent ou un mélange des ces groupes alkyle.

11. Procédé selon l'une quelconque des revendications 1 à 8, **caractérisé en ce que** R₃ représente un groupe alkylène en C₂ à C₈ ou un groupe alkylène en C₄ à C₁₂ interrompu par un atome d'oxygène, de préférence un groupe dérivé d'un diol en éliminant les deux groupes OH, de préférence dérivé d'alkylèneglycols, de préférence de 1,2-éthanediol, de 1,3-propanediol, de 1,4-butanediol, de 1,5-pentanediol, de 1,6-hexanediol, de 1,7-heptanediol, de 1,8-octanediol ; de polyalkylèneglycols, de préférence de diéthylèneglycol, de triéthylèneglycol et de tétraéthylèneglycol, de dipropylèneglycol, de tripropylèneglycol ; de glycérine, de pentaérythritol ou de polyols analogues ; de composés polyhydroxyle éthérifiés, de préférence de polyglycérine ou de pentaérythritol.

12. Procédé selon l'une quelconque des revendications 1 à 9, **caractérisé en ce que** R₄ représente un groupe alkylène dérivé d'une diamine en éliminant les deux groupes H₂N, lequel est éventuellement interrompu par un groupe -NR₅-, de préférence par -NH-, et lequel est dérivé de préférence d'une alkylènediamine, de préférence de 1,2-éthylènediamine ou de 1,2-propylènediamine ou d'une dialkylènediamine, de préférence de di(éthylènediamine) ou de di(propylènediamine).

13. Procédé selon l'une quelconque des revendications 1 à 8, **caractérisé en ce que** R₃ représente un groupe alkyle-alkylène ramifié dérivé d'un alcool trivalent (polyol) en éliminant les groupes OH, de préférence en éliminant les groupes OH de 1,1,1-trishydroxyméthyléthane (triméthyloléthane) et de 1,1,1-tris-hydroxyméthylpropane (triméthylolpropane).

14. Procédé selon l'une quelconque des revendications 1 à 8, **caractérisé en ce que** R₃ représente un groupe alkyle-alkylène ramifié dérivé d'un tétraol en éliminant les groupes OH, de préférence un groupe méthane-tétraméthylène.

15. Procédé selon l'une quelconque des revendications 1 à 11, **caractérisé en ce que**, dans le composé de formule (I), R₁ représente un groupe tert-butyle et R₂ représente un groupe méthyle ou tert-butyle, et R₃ représente un groupe n-octadécyle, un mélange de groupes alkyle supérieurs contenant 8 à 30 atomes de carbone ou un groupe dérivé de 1,6-hexanediol, de triéthylèneglycol, de pentaérythritol, de 1,1,1-trishyroxyméthyléthane ou de 1,1,1-tris-hydroxyméthylpropane.

16. Procédé selon l'une quelconque des revendications 1 à 11, **caractérisé en ce que**, dans le composé de formule (I), R₁ représente un groupe tert-butyle, et R₂ représente un groupe méthyle ou tert-butyle, et R₄ représente un groupe dérivé de 1,2-éthylènediamine ou de 1,2-propylènediamine ou de di(éthylènediamine) ou de di(propylènediamine).

17. Procédé selon l'une quelconque des revendications 1 à 16, **caractérisé en ce que** les composés de formules (II) et (III) sont chauffés conjointement avec le catalyseur dans des conditions inertes, à une température de 90°C à 120°C, jusqu'à ce qu'il se forme une masse fondue, et la réaction de transestérification est ensuite réalisée sous pression réduite dans la gamme de 0,1 à 200 mbars et à haute température dans la gamme de 140°C à 220°C.

18. Procédé selon l'une quelconque des revendications 1 à 17, **caractérisé en ce que** le rapport du composé de formule (II) au composé de formule (III) [calculé en équivalents molaires] est dans la gamme de 0,8:1 à 1,5:1, de préférence dans la gamme de 1:1 à 1,2:1, et de manière plus particulièrement préférée il est compris entre 1,05:1 et 1,15:1.

19. Procédé selon l'une quelconque des revendications 1 à 18, **caractérisé en ce que** le produit de formule (I) est amené à se figer ou à cristalliser par refroidissement, avec ou sans germe de cristallisation, et il est transformé directement dans la forme commercialisable sans étape de purification supplémentaire.

20. Utilisation de sels alcalins d'un acide carboxylique organique, ou de mélanges de tels sels alcalins, selon l'une quelconque des revendications 2 à 5, de préférence l'acétate de sodium, l'acétate de potassium, l'acétate de lithium, le formiate de sodium, le formiate de potassium et le formiate de lithium ou un mélange des ces composés, en tant que catalyseur pour la production de composés de formule (I) selon l'une quelconque des revendications 1 à 19.

21. Utilisation selon la revendication 20, **caractérisée en ce que** le catalyseur est présent à raison de 0,05% à 5% en moles, de préférence de 0,05% à 3% en moles, de préférence de 0,1% à 1% en moles, par rapport à la quantité molaire du composé de formule (I) à faire réagir.

22. Composés de formule (I), dans laquelle R₃ représente un groupe R₄-[NR₅-CₘH₂ₘ-]ₚ, de préférence R₄-[NH-CₘH₂ₘ-]ₚ, où R₄ représente un groupe dérivé de 1,2-éthylènediamine ou de 1,2-propylènediamine ou de di(éthylènediamine) ou de di(propylènediamine), et R₁, R₂ et m ont les significations selon la revendication 1.
